# FASCICULE DE BREVET EUROPEEN

(11) **EP 1 240 875 B1**
(45) Date de publication et mention de la délivrance du brevet: **05.10.2005**
(21) Numéro de dépôt: 02290631.7
(22) Date de dépôt: 13.03.2002
(51) Int. Cl.: A61B 17/70

(54) **Organe d'ancrage rachidien avec une bague de sécurité**
Wirbelsäulenanker mit Sicherungsband
Spinal anchor with locking collar

(30) Priorité: 15.03.2001 FR 0103515
(43) Date de publication de la demande: 18.09.2002
(73) Titulaire: Stryker Spine, 33610 Cestas (FR)
(72) Inventeur: Saint Martin, Pierre Henri, 33700 Merignac (FR)
(74) Mandataire: Le Forestier, Eric

(56) Documents cités:
- WO-A-01/15612
- DE-U- 29 903 342
- US-A- 5 466 237
- US-A- 6 053 917

## Description

L'invention concerne les système d'ostéosynthèse, notamment pour la chirurgie de la colonne vertébrale.

Le document WO 98/12 976 présente un système d'ostéosynthèse rachidien comprenant un organe d'ancrage de type vis polyaxiale dont le blocage en position s'effectue par appui de la tige de liaison sur une couronne dont la surface inférieure arrondie vient en appui de manière complémentaire sur la tête sphérique de la vis osseuse logée dans le fond d'un logement aménagé dans un connecteur. Un tel système, sur lequel le préambule de la revendication 1 est fondé, nécessite un effort très important d'appui de la tige sur la couronne pour que la pression surfacique entre la couronne et la tête de vis soit suffisante pour éviter tout mouvement de l'un par rapport à l'autre, mouvement qui aurait pour conséquence de créer une instabilité néfaste à l'ostéosynthèse recherchée.

Un but de l'invention est de fournir un dispositif de blocage en position plus sûr, pour un même effort de serrage.

Pour cela, on prévoit, selon l'invention, un ensemble d'ostéosynthèse rachidienne tel que défini dans la revendication 1.

Ainsi, lors du verrouillage du système d'ostéosynthèse, l'appui de l'organe de liaison sur l bague oblige cette dernière à venir appuyer sur ladite partie de l'organe d'ancrage osseux pour bloquer celui-ci en position au sein du connecteur, et l'appui simultané de l'organe de liaison sur ladite partie de l'organe d'ancrage vient renforcer le blocage précédent, ce qui le sécurise tout en conservant le même effort de serrage pour le verrouillage.

Des aspects préférés mais non limitatifs de cet ensemble sont définis dans les revendications dépendantes.

D'autres caractéristiques et avantages de l'invention apparaîtront lors de la description suivante d'un mode préféré de réalisation. Aux dessins annexés :
- la figure 1 est une vue en perspective du mode préféré de réalisation de l'invention ;
- la figure 2 est une vue en coupe selon le plan II-II du mode de réalisation de la figure 1 ;
- la figure 3 est une vue en perspective de la bague du mode de réalisation préféré ;
- la figure 4 est une vue en perspective du mode de réalisation de la figure 1 avant la mise en place de l'organe de liaison ; et
- la figure 5 est une vue en coupe selon le plan V-V du mode de réalisation de la figure 4.

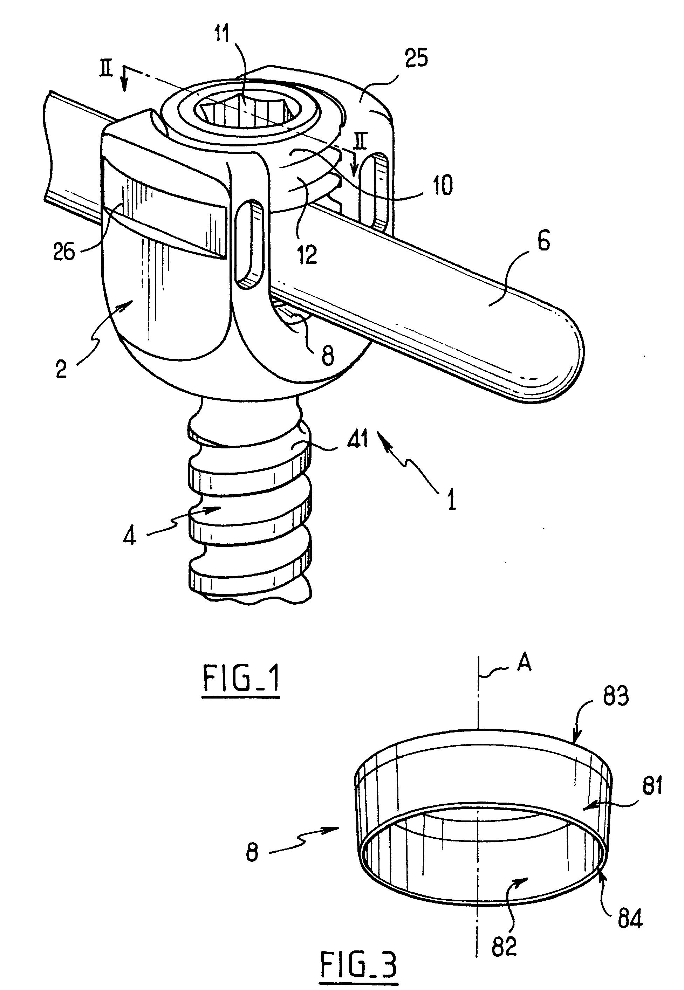

En référence aux différentes figures 1 à 5, un mode préféré va être décrit. L'ensemble pour ostéosynthèse de la colonne vertébrale 1 comprend, ici, un connecteur 2, un organe de liaison 6 et des moyens d'ancrage 4. Ici, l'organe de liaison 6 est une tige d'ostéosynthèse et les moyens d'ancrage 4 sont formés par une vis pédiculaire. L'ensemble 1 comprend en outre une bague 8 ainsi qu'un verrou 10 apte à verrouiller l'ensemble 1 en position.

Le connecteur 2 comporte une ouverture dite en « U » 21 formant la partie supérieure du connecteur 2. Cette ouverture en « U » 21 est délimitée par deux branches 25 et 26 qui s'étendent de manière sensiblement parallèle l'une par rapport à l'autre. Les faces internes des branches 25 et 26 qui s'étendent en regard l'une de l'autre comportent un filetage 24. D'autre part, le connecteur 2, dans sa partie inférieure, comprend un logement interne 22 présentant une paroi 23. La partie supérieure du logement interne 22 débouche dans le fond de l'ouverture en « U » 21, et sa partie inférieure débouche de manière opposée sur une face inférieure 27 du connecteur 2. Du coté de la face inférieure 27, la paroi 23 présente une section conique agencée de manière à ce que l'ouverture au niveau de la face inférieure 27 soit plus petite que l'ouverture au niveau du fond de l'ouverture en « U » 21.

Le verrou 10 comprend des moyens de mise en oeuvre 11 qui se présentent ici sous la forme d'un orifice traversant 11 présentant une empreinte hexagonale. Cette empreinte hexagonale est apte à recevoir un embout hexagonal adapté d'un tournevis pour sa mise en oeuvre. D'autre part, le verrou 10 comprend sur sa paroi latérale extérieure un filetage 12 complémentaire du filetage 24 du connecteur 2 entre les branches 25 et 26 duquel il est apte à être reçu.

Les moyens d'ancrage 4 se présentent ici sous la forme d'une vis pédiculaire comportant une partie d'ancrage 41 présentant un filetage osseux, surmontée d'une tête 42 qui est ici sensiblement sphérique. La tête 42 présente une première surface sphérique 43 et, formant le sommet, une seconde surface sphérique 44 dont le diamètre est inférieur au diamètre de la surface sphérique 43 mais de même centre que celle-ci.

On pourra trouver des systèmes d'ostéosynthèse similaires dans le document EP-0 613 664.

La bague 8 est de forme annulaire et présente une première face 82 délimitant la paroi interne de la bague, une seconde face 81 délimitant la paroi externe de la bague ainsi que des bords supérieur 83 et inférieur 84 perpendiculaires à l'axe de révolution géométrique A de la bague 8. Les faces 81 et 82 sont coaxiales et préférentiellement de forme conique. Leurs génératrices respectives ne sont pas parallèles entre elles. Ainsi, les faces sont agencées l'une par rapport à l'autre de manière à ce que l'épaisseur de la bague 2 au niveau du bord supérieur 83 est supérieure à l'épaisseur de la bague 8 au niveau du bord inférieur 84. La section de la bague présente ainsi une forme de coin, donnant une forme en biseau de la bague 8. Cependant, l'une des génératrices des faces 81 et 82 peut être sensiblement parallèle à l'axe de révolution A.

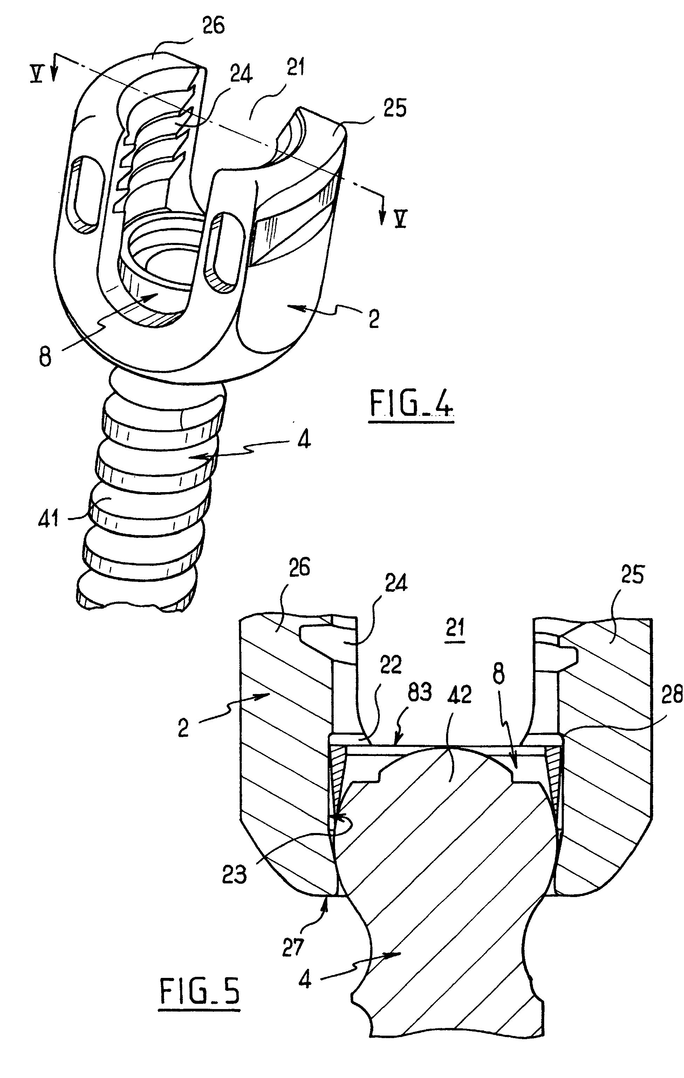

Avant utilisation par un chirurgien, le connecteur 2, les moyens d'ancrage 4 ainsi que la bague 8 sont montés ensemble. La tête 42 des moyens d'ancrage est insérée au sein du logement interne 22 du connecteur 2. Puis, la bague 8 est elle-même insérée au sein du logement interne 22 du connecteur 2. Ainsi, la tête 42 des moyens d'ancrage 4 se trouve retenue prisonnière au sein du logement interne 22 à l'encontre de sa sortie par le haut, du fait de la présence au sein du logement interne 22 de la bague 8, elle-même retenue prisonnière comme on le verra plus loin. La tête 42 est retenue à l'encontre de sa sortie par le bas, du fait de la présence de la section conique de la paroi 23 du logement interne 22, qui présente une ouverture au niveau de la face inférieure 27 du connecteur 2 dont les dimensions sont inférieures au diamètre de la surface 43 de la tête 42. De plus, la bague 8 est retenue prisonnière par des moyens de retenue 28 présents au sein du logement interne 22. Ici, les moyens de retenue 28 viennent de la différence de dimension entre le logement interne 22 et l'ouverture en U 21, différence formant un rebord sur lequel vient en butée par le bas le bord supérieur 83 de la bague 8. Cet assemblage est illustré aux figures 4 et 5.

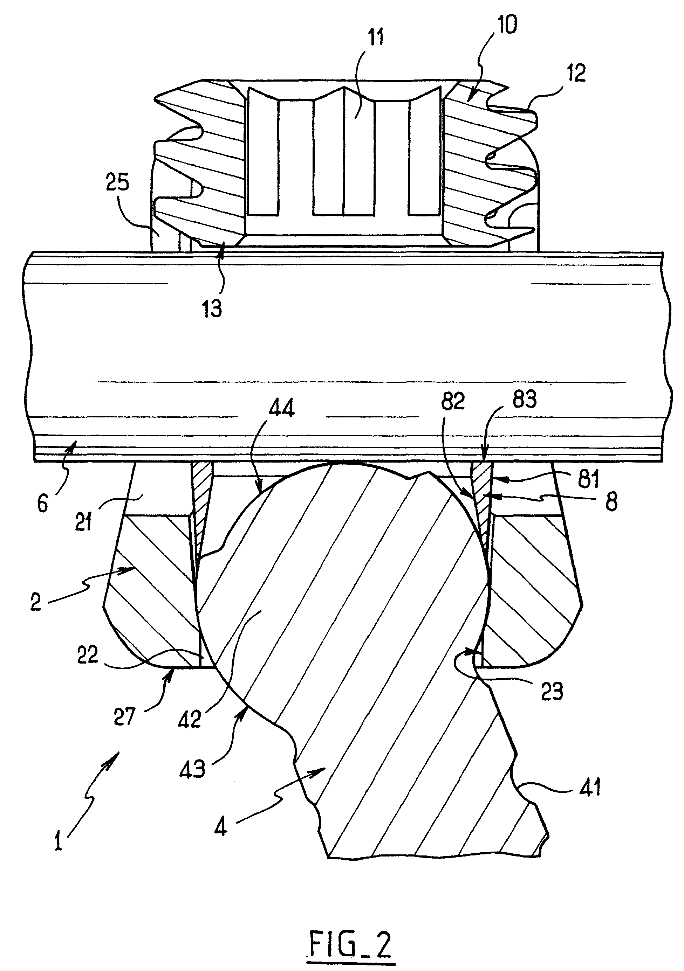

Lors de l'utilisation au cours d'une opération chirurgicale, le chirurgien met en place dans le pédicule un assemblage tel que décrit précédemment. Puis il met en place l'organe de liaison 6 en l'insérant dans l'ouverture en « U » 21 du connecteur 2. Puis il met en place le verrou 10 entre les branches 25 et 26, en mettant en prise le filetage 12 du verrou 10 avec le filetage complémentaire 24 du connecteur 2. Il met en oeuvre, par l'empreinte hexagonale 11, le verrou 10, de manière à ce que la face inférieure 13 du verrou 10 vienne en contact avec l'organe de liaison 6.
En continuant de visser le verrou 10 entre les branches 25 et 26, le chirurgien va faire exercer un effort par le verrou 10 sur l'organe de liaison 6, ce qui va pousser l'organe de liaison 6 jusqu'à ce que ce dernier vienne en appui sur le bord supérieure 83 de la bague 8.
Le verrouillage se poursuivant, la bague 8 glisse alors le long de la paroi 23 du logement interne 22 jusqu'à ce que la face 82 de la bague 8 vienne en contact avec la surface 43 de la tête 42 des moyens d'ancrage 4. La surface 43 est elle-même en contact avec la section conique de la paroi 23 du logement interne 22 du connecteur 2. Le système se trouve de ce fait dans une situation telle qu'illustrée à la figure 2.
Lors du verrouillage final qui va permettre le blocage en position de l'ensemble, l'effort de serrage imposé par le chirurgien par l'intermédiaire du verrou 10 va permettre de faire glisser la bague 8 sur la tête 42. Pour cela, la face 82 va glisser sur la surface 43, obligeant la bague 8 à s'ouvrir par déformation jusqu'à ce que la face 81 de la bague 8 vienne en contact sur toute ou partie de sa surface avec la paroi 23 du logement interne 22 du connecteur 2. A ce moment, l'organe de liaison 6 vient appuyer en un point de la surface sphérique 44. Ainsi, la tête 42 est bloquée en position, d'une part par la bague 8 et d'autre part, par l'organe de liaison 6 directement. On a ainsi un appui dit trois points, deux points diamétralement opposés au contact du bord 83 de la bague 8 avec l'organe de liaison 6 et un point supplémentaire au niveau du contact de l'organe de liaison 6 avec la surface 44 de la tête 42 des moyens d'ancrage 4.

Bien entendu, on pourra apporter à l'invention de nombreuses modifications sans sortir du cadre de celle-ci.
Par exemple, la bague 8 pourra comporter au moins une fente.
La fente pourra être agencée de sorte que la bague forme un anneau non fermé.

La forme en biseau due à la forme en coin de la section de la bague, au lieu d'être continue sur toute sa circonférence, pourra être constituée d'une pluralité de secteurs séparés par des fentes pour former une structure dite « en parapluie ».
Ces différentes modifications permettent une déformation plus facile de la bague 8. Ceci a pour conséquence de faciliter l'introduction de la bague 8 au sein du logement interne 22 du connecteur 2, d'une part, et, d'autre part, le verrouillage final lors de l'utilisation en cours d'opération chirurgicale.

## Revendications

1. Ensemble d'ostéosynthèse rachidienne (1) comprenant un connecteur (2), un organe d'ancrage osseux (4) dont une partie (42) est apte à être reçue dans le connecteur, un organe de liaison (6) également apte à être reçu dans le connecteur, une bague (8), apte à venir en contact avec ladite partie de l'organe d'ancrage osseux (4) pour bloquer celui-ci en position au sein du connecteur (2), et un organe de verrouillage (10) apte à solliciter l'organe de liaison (6) en direction de ladite partie (42) de l'organe d'ancrage osseux et de ladite bague (8), **caractérisé en ce que** lors d'une telle sollicitation, l'organe de liaison est apte à venir en appui simultanément sur la bague et sur ladite partie de l'organe d'ancrage osseux.

2. Ensemble selon la revendication 1, **caractérisé en ce que** la bague présente au moins une face conique (81,82).

3. Ensemble selon la revendication 1 ou 2, **caractérisé en ce que** la bague présente une face (82) apte à venir en contact avec ladite partie de l'organe d'ancrage osseux.

4. Ensemble selon l'une des revendications 1 à 3 **caractérisé en ce que** la bague présente une face (81) apte à venir en contact avec une paroi (23) du connecteur.

5. Ensemble selon les revendications 3 et 4, **caractérisé en ce que** les faces (81,82) sont coaxiales.

6. Ensemble selon l'une des revendications 1 à 5, **caractérisé en ce que** la bague présente un bord supérieur (83) plan, perpendiculaire à un axe (A) de la bague et apte à venir en contact avec l'organe de liaison.

7. Ensemble selon l'une des revendications 1 à 6, **caractérisé en ce que** la bague présente un bord inférieur (84) plan et perpendiculaire à un axe (A) de la bague.

8. Ensemble selon l'une des revendications 1 à 7, **caractérisé en ce que** la bague est apte à s'étendre entre la paroi (23) et ladite partie (42) de l'organe d'ancrage lorsque l'organe de liaison réalise ledit appui.

9. Ensemble selon l'une des revendications précédentes, **caractérisé en ce que** la bague est apte à se déformer lorsque l'organe de liaison réalise ledit appui, par référence à une forme de la bague au repos.

10. Ensemble selon l'une des revendications 1 à 9, **caractérisé en ce que** la bague présente une épaisseur de paroi qui varie suivant une hauteur.

11. Ensemble selon l'une des revendications précédentes, **caractérisé en ce que** la bague comprend une fente.

12. Ensemble selon la revendication 11, **caractérisé en ce que** la fente est agencée de sorte que la bague forme un anneau non fermé.

13. Ensemble selon la revendication 11, **caractérisé en ce que** la bague comprend une pluralité de fentes uniformément réparties sur une circonférence de la bague.

14. Ensemble selon l'une des revendications 1 à 13, **caractérisé en ce que** ladite partie de l'organe d'ancrage comprend une tête (42) présentant une face (43,44) sensiblement sphérique.

15. Ensemble selon la revendication 14, **caractérisé en ce que** la tête présente des première (43) et seconde (44) faces sphériques de même centre et de diamètres sensiblement différents.

16. Ensemble selon l'une des revendications précédentes, **caractérisé en ce que** l'organe d'ancrage forme une vis polyaxiale.

17. Ensemble selon l'une des revendications précédentes, **caractérisé en ce que** l'organe de verrouillage comprend un verrou (10) vissé dans le connecteur.

18. Système d'ostéosynthèse **caractérisé en ce qu'**il comprend un ensemble selon l'une des revendications 1 à 17.

## Patentansprüche

1. Wirbelsäulen-Osteosyntheseanordnung (1), umfassend ein Anschlußstück (2), ein Knochen-Verankerungsorgan (4), dessen einer Teil (42) dazu geeignet ist, im Anschlußstück aufgenommen zu werden, ein Verbindungsorgan (6), das ebenfalls dazu geeignet ist, im Anschlußstück aufgenommen zu werden, einen Ring (8), der dazu geeignet ist, in Berührung mit dem genannten Teil des Knochen-Verankerungsorganes (4) zu gelangen, um dieses in seiner Lage inmitten des Anschlußstücks (2) zu blockieren, und ein Verriegelungsorgan (10), das dazu geeignet ist, das Verbindungsorgan(6) in Richtung des genannten Teils (42) des Verankerungsorgans und des genannten Rings (8) zu belasten, **dadurch gekennzeichnet, daß** das Verbindungsorgan dazu geeignet ist, während einer solchen Belastung gleichzeitig auf dem Ring und auf dem genannten Teil des Knochen-Verankerungsorgans in Auflage zu gelangen.

2. Anordnung nach Anspruch 1, **dadurch gekennzeichnet, daß** der Ring mindestens eine konische Fläche (81, 82) aufweist.

3. Anordnung nach Anspruch 1 oder 2, **dadurch gekennzeichnet, daß** der Ring eine Fläche (82) aufweist, die dazu geeignet ist, in Berührung mit dem genannten Teil des Knochen-Verankerungsorgans zu gelangen.

4. Anordnung nach einem der Ansprüche 1 bis 3, **dadurch gekennzeichnet, daß** der Ring eine Fläche (81) aufweist, die dazu geeignet ist, in Berührung mit einer Wand (23) des Anschlußstücks zu gelangen.

5. Anordnung nach den Ansprüchen 3 und 4, **dadurch gekennzeichnet, daß** die Flächen (81, 82) koaxial sind.

6. Anordnung nach einem der Ansprüche 1 bis 5, **dadurch gekennzeichnet, daß** der Ring einen oberen, ebenen Rand (83) aufweist, der senkrecht zu einer Achse (A) des Rings steht und dazu geeignet ist, mit dem Verbindungsorgan in Berührung zu treten.

7. Anordnung nach einem der Ansprüche 1 bis 6, **dadurch gekennzeichnet, daß** der Ring einen unteren Rand (84) aufweist, der eben ist und senkrecht zu einer Achse (A) des Rings steht.

8. Anordnung nach einem der Ansprüche 1 bis 7, **dadurch gekennzeichnet, daß** der Ring dazu geeignet ist, sich zwischen der Wand (23) und dem genannten Teil (42) des Verankerungsorgans zu erstrecken, wenn das Verbindungsorgan die genannte Auflage herstellt.

9. Anordnung nach einem der vorhergehenden Ansprüche, **dadurch gekennzeichnet, daß** der Ring dazu geeignet ist, sich zu verformen, wenn das Verbindungsorgan die genannte Auflage herstellt, bezogen auf eine Form des Rings in Ruhelage.

10. Anordnung nach einem der Ansprüche 1 bis 9, **dadurch gekennzeichnet, daß** der Ring eine Wandstärke aufweist, die sich nach der Höhe ändert.

11. Anordnung nach einem der vorangehenden Ansprüche, **dadurch gekennzeichnet, daß** der Ring einen Schlitz aufweist.

12. Anordnung nach Anspruch 11, **dadurch gekennzeichnet, daß** der Schlitz derart ausgebildet ist, daß der Ring einen nicht geschlossenen Kreisring bildet.

13. Anordnung nach Anspruch 11, **dadurch gekennzeichnet, daß** der Ring eine Vielzahl von Schlitzen aufweist, die über einen Umfang des Rings gleichmäßig verteilt sind.

14. Anordnung nach einem der Ansprüche 1 bis 13, **dadurch gekennzeichnet, daß** der genannte Teil des Verankerungsorgans einen Kopf (42) umfaßt, der eine im wesentlichen kugelige Fläche (43, 44) aufweist.

15. Anordnung nach Anspruch 14, **dadurch gekennzeichnet, daß** der Kopf eine erste (43) und eine zweite (44) Fläche aufweist, die zur selben Mitte kugelig sind und im wesentlichen unterschiedliche Durchmesser aufweisen.

16. Anordnung nach einem der vorhergehenden Ansprüche, **dadurch gekennzeichnet, daß** das Verankerungsorgan eine polyaxiale Schraube bildet.

17. Anordnung nach einem der vorhergehenden Ansprüche, **dadurch gekennzeichnet, daß** das Verriegelungsorgan ein Sperrglied (10) umfaßt, das in das Anschlußstück eingeschraubt ist.

18. Osteosynthesesystem, **dadurch gekennzeichnet, daß** es eine Anordnung nach einem der Ansprüche 1 bis 17 umfaßt.

## Claims

1. Spinal osteosynthesis assembly (1) comprising a connector (2), a bone anchoring member (4), part (42) of which is capable of being received in the connector, a connecting member (6) which is also capable of being received in the connector, a ring (8) capable of coming into contact with the said part of the bone anchoring member (4) so as to lock the latter in position within the connector (2), and a locking member (10) capable of urging the connecting member (6) towards the said part (42) of the bone anchoring member and the said ring (8), **characterized in that** during such urging the connecting member is capable of bearing simultaneously on the ring and on the said part of the bone anchoring member.

2. Assembly according to Claim 1, **characterized in that** the ring has at least one conical face (81, 82).

3. Assembly according to Claim 1 or 2, **characterized in that** the ring has a face (82) capable of coming into contact with the said part of the bone acnhoring member.

4. Assembly according to one of Claims 1 to 3, **characterized in that** the ring has a face (81) capable of coming into contact with a wall (23) of the connector.

5. Assembly according to Claims 3 and 4,
**characterized in that** the faces (81, 82) are coaxial.

6. Assembly according to one of Claims 1 to 5, **characterized in that** the ring has a flat upper edge (83) perpendicular to an axis (A) of the ring and capable of coming into contact with the connecting member.

7. Assembly according to one of Claims 1 to 6, **characterized in that** the ring has a flat lower edge (84) perpendicular to an axis (A) of the ring.

8. Assembly according to one of Claims 1 to 7, **characterized in that** the ring is capable of extending between the wall (23) and the said part (42) of the anchoring member when the connecting member bears as mentioned.

9. Assembly according to one of the preceding claims, **characterized in that** the ring is capable of being deformed when the connecting member realizes said bearing, compared to the shape of the ring at rest.

10. Assembly according to one of Claims 1 to 9, **characterized in that** the ring has a wall thickness which varies according to a height.

11. Assembly according to one of the preceding claims, **characterized in that** the ring comprises a slot.

12. Assembly according to Claim 11, **characterized in that** the slot is arranged in such a way that the ring forms a non-closed annulus.

13. Assembly according to Claim 11, **characterized in that** the ring comprises a number of slots distributed uniformly about a circumference of the ring.

14. Assembly according to one of Claims 1 to 13, **characterized in that** the said part of the anchoring member comprises a head (42) having a roughly spherical face (43, 44).

15. Assembly according to Claim 14, **characterized in that** the head has a first spherical face (43) and a second spherical face (44) which have the same centre and significantly different diameters.

16. Assembly according to one of the preceding claims, **characterized in that** the anchoring member forms a polyaxial screw.

17. Assembly according to one of the preceding claims, **characterized in that** the locking member comprises a lock (10) screwed into the connector.

18. Osteosynthesis system **characterized in that** it comprises an assembly according to one of Claims 1 to 17.
